# EUROPEAN PATENT APPLICATION

(11) **EP 4 596 548 A1**
(43) Date of publication of application: **06.08.2025**
(21) Application number: 24155686.9
(22) Date of filing: 05.02.2024
(51) Int. Cl.: C07D 471/04, A61K 31/4353, A61P 25/28, A61P 35/00

(54) **NURR1 MODULATORS**

(71) Applicant: Ludwig-Maximilians-Universität, 80539 München (DE)
(72) Inventor: MERK, Daniel, 80333 München (DE); SAI, Nhat Minh, 80807 München (DE)
(74) Representative: Weickmann & Weickmann PartmbB

(57) **Abstract**

The present invention relates to novel imidazopyridine compounds as modulators of the transcription factor Nuclear receptor related 1 (Nurr1, NR4A2) in medicine, particularly in the prevention and/or treatment of a degenerative and/or inflammatory disease.

## Description

The present invention relates to novel imidazopyridine compounds as modulators of the transcription factor Nuclear receptor related 1 (Nurr1, NR4A2) in medicine, particularly in the prevention and/or treatment of a degenerative disease or an inflammatory disease and/or a hyperproliferative disease such as cancer.

### BACKGROUND

Nurr1 is a ligand-activated transcription factor mainly expressed in neurons and immune cells of the brain ¹⁻³. The receptor is critically involved in the regulation of (dopaminergic) neuron development and maintenance as well as inflammatory processes ³⁻⁶. Observations of diminished Nurr1 levels in patients ⁷ and animal models ⁸⁻¹⁰ of Alzheimer's (AD) and Parkinson's disease (PD) underline therapeutic potential of Nurr1 modulation in neurodegenerative diseases. Moreover, recent findings suggest a protective and anti-inflammatory role of Nurr1 in retinal pigment epithelial cells in the eye with possible therapeutic relevance in age-related macular degeneration ¹¹. Pharmacological modulation of Nurr1 activity may therefore offer new therapeutic options in various degenerative diseases and potent Nurr1 modulators are needed 12

Nurr1 acts as a monomer, homodimer or heterodimer and has constitutive transcriptional activator activity also in absence of ligands but can be modulated by agonists and inverse agonists in a bidirectional fashion ¹³⁻¹⁵. The dopamine metabolite 5,6-dihydroxyindole (DHI) ¹⁶, poly-unsaturated fatty acids ¹⁷ and the prostaglandinsAand E ¹⁸ have been identified as natural Nurr1 ligands. A few synthetic Nurr1 ligand chemotypes have been recently identified ¹⁹⁻²³ among which the antimalarial amodiaquine (AQ, **1**; EC₅₀ ~20 µM) ²⁴ was the first validated Nurr1 agonist and emerged as a valuable early tool to study therapeutic effects of Nurr1 activation. AQ treatment counteracted neuroinflammation and ameliorated behavioral deficits in a PD model ²⁴ and reduced neuronal loss and amyloid beta deposition in an AD model ²⁵. Structural optimization efforts have recently yielded the AQ descendant **2** which exhibits improved Nurr1 agonist potency and mediated protective effects in a PD model ²⁶.

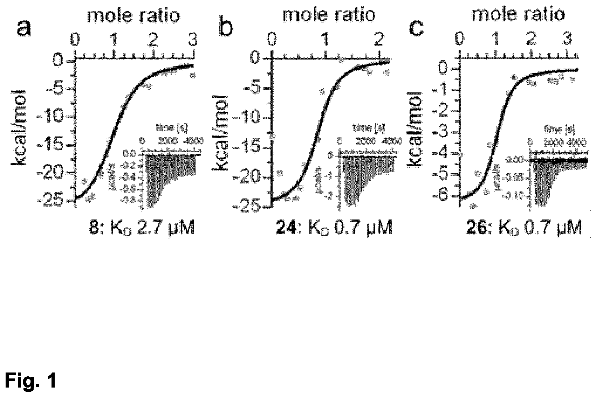

These promising observations suggest Nurr1 ligands derived from AQ (**1**) as attractive tools and eventually drug candidates (Fig. 1). We have previously discovered that AQ (**1**) can be simplified to its fragment-like 7-chloroquinolin-4-amine (**3**) substructure without fully losing Nurr1 agonism (EC₅₀ 259 µM) ²⁷. Notably, the Nurr1 agonism of the fragment **3** could be tuned by minor structural modifications to the substantially more potent Nurr1 agonist 8-chloro-2-methylquinolin-4-amine (**4**, EC₅₀ 17 µM) ²⁷. Additionally, we identified a 5-(4-chlorophenyl) furan-2-carboxamide motif (**5**, EC₅₀ 3.0 µM) ²⁸ as a replacement for the unfavorable 4-aminophenol residue of AQ (**1**).

### Compounds (1) - (5) are depicted in the following Chart 1:

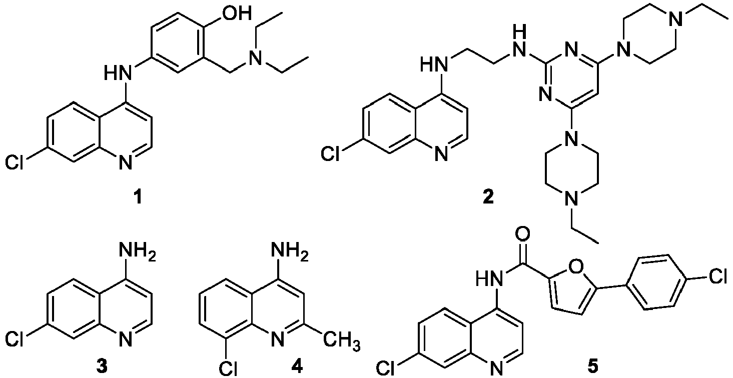

It was an object of the present invention to provide novel and more active Nurr1 modulators.

### SUMMARY OF THE INVENTION

A first aspect of the present invention relates to a compound of formula (I) 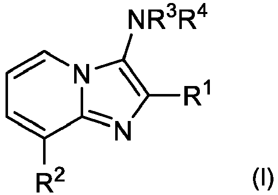 wherein
- R¹: is selected from -H, -OH, -C₁-C₄ alkyl optionally substituted, -C₂-C₄ alkenyl optionally substituted, -C₂-C₄ alkynyl optionally substituted, -C₃-C₈ cycloalkyl optionally substituted, -OC₁-C₄ alkyl optionally substituted, -OC₂-C₄ alkenyl optionally substituted, -OC₂-C₄ alkynyl optionally substituted, -OC₃-C₈ cycloalkyl optionally substituted, phenyl optionally substituted, heteroaryl optionally substituted, -O-phenyl optionally substituted or -O-heteroaryl optionally substituted,
- R²: is selected from halo, -C₁-C₄ alkyl optionally substituted, -C₂-C₄ alkenyl optionally substituted, C₂-C₄ alkynyl optionally substituted -OC₁-C₄ alkyl optionally substituted, -OC₂-C₄ alkenyl optionally substituted, -OC₂-C₄ alkynyl optionally substituted, -C₃-C₈ cycloalkyl optionally substituted, or -OC₃-C₈ cycloalkyl optionally substituted,
- R³: is selected from H or -CH₃ optionally substituted,
- R⁴: is selected from H, -CH₃ optionally substituted or -R⁵,
- R⁵: is 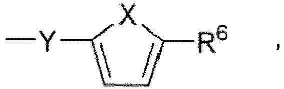
- X: is selected from O, S or NR¹¹,
- Y: is selected from -C(O)- or C₁-C₃- alkylene,
- R⁶: is selected from phenyl, heteroaryl, -C₃-C₈ cycloalkyl, -C₁-C₆ alkyl, -C₂-C₆ alkenyl, -C₂-C₆ alkynyl, wherein each of said residues is optionally substituted with -R⁷, -OR⁸, -NR⁹R¹⁰ and/or halo;
- R⁷: is selected from -H, halo, -C₁-C₆ alkyl optionally substituted, -C₂-C₆ alkenyl optionally substituted, -C₂-C₆ alkynyl optionally substituted, or -C₃-C₈ cycloalkyl optionally substituted,
- R⁸: is selected from H, halo, C₁-C₆ alkyl optionally substituted, -C₂-C₆ alkenyl optionally substituted, -C₂-C₆ alkynyl optionally substituted or C₃-C₈ cycloalkyl optionally substituted,
- R⁹ and R¹⁰: are independently selected from H, halo, C₁-C₆ alkyl optionally substituted, -C₂-C₆ alkenyl optionally substituted, -C₂-C₆ alkynyl optionally substituted or C₃-C₈ cycloalkyl optionally substituted or wherein R⁹ and R¹⁰ may form a cyclic system, and
- R¹¹: is selected from H, C₁-C₆ alkyl optionally substituted, -C₂-C₆ alkenyl optionally substituted, -C₂-C₆ alkynyl optionally substituted or C₃-C₈ cycloalkyl optionally substituted.

A further aspect of the present invention relates to the use of the compound of formula (I) or a salt or solvate thereof in medicine, e.g., in human or veterinary medicine.

A further aspect of the present invention relates to a pharmaceutical composition comprising the compound of formula (I) or a salt or solvate thereof as an active agent and a pharmaceutically acceptable carrier.

A further aspect of the present invention relates to the use of the compound of formula (I) or a salt or solvate thereof in in the prevention and/or treatment of a disease caused by and/or associated with insufficient Nurr1 activity.

A further aspect of the present invention relates to the use of the compound of formula (I) or a salt or solvate thereof in in the prevention and/or treatment of a degenerative disease, an inflammatory disease and/or a hyperproliferative disease such as cancer.

A further aspect relates to a method of preventing and/or treating a disease caused by and/or associated with insufficient Nurr1 activity comprising administering to a subject, e.g., a human subject, in need thereof an effective amount of a compound of formula (I).

A further aspect relates to method of preventing and/or treating a degenerative disease, an inflammatory disease and/or a hyperproliferative disease such as cancer comprising administering to a subject, e.g., a human subject, in need thereof an effective amount of a compound of formula (I).

### DETAILED DESCRIPTION

The present invention relates to a novel class of Nurr1 modulators with substantially enhanced potency. These compounds are useful as medicaments and high-quality chemical tools.

In certain embodiments, the compounds are Nurr1 agonists, i.e. the compounds are activators of Nurr1. Activation of Nurr1 may be determined quantitatively in a Gal4-Nurr1 hybrid reporter assay or reporter gene assays for full-length Nurr1 and the response elements NurRE, NBRE and DR5 as described herein.

In certain embodiments, a compound of the present invention has an EC₅₀ for Nurr1 activation of about 1 µM or less, particularly of about 0.5 µM or less and more particularly of about 0.01 µM or less.

In particular embodiments, a compound of the present invention selectively activates a Nurr1 dimer, e.g., a Nurr1 homodimer and a Nurr1-RXR heterodimer but is inactive on a Nurr1 monomer.

In further embodiments, the compounds are inverse Nurr1 agonists, i.e. the compounds are inhibitors of Nurr1 activity. Inhibition of Nurr1 may be determined quantitatively in a Gal4-Nurr1 hybrid reporter assay or reporter gene assays for full-length Nurr1 and the response elements NurRE, NBRE and DR5 as described herein.

In further embodiments, a compound of the present invention has an IC₅₀ for Nurr1 inhibition of about 1 µM or less, particularly of about 0.5 µM or less and more particularly of about 0.01 µM or less.

The present invention relates to compounds of formula (I) and salts or solvates thereof: 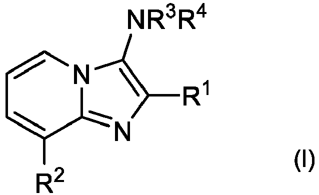 as described herein.

The definitions of atoms in the compounds of formula (I) encompasses all possible isotopes including stable and unstable isotopes. For example, the term "H" encompasses hydrogen isotopes including deuterium.

In individual residues of compounds of formula (I) the following general definitions apply:
The term "optionally substituted" includes "unsubstituted" and "substituted", e.g., "mono- and polysubstituted".

The terms "alkyl", "alkenyl" and "alkynyl" include linear and branched alkyl, alkenyl and alkynyl residues which are optionally substituted.

The term "cyclic system" relates to mono- or polycyclic ring system, particularly a 3- to 8-membered ring, e.g., a saturated, unsaturated, spiro- or aromatic ring which is optionally substituted. Preferred substituents of phenyl include -OH, halo, -C₁-C₆ alkyl optionally substituted, -C₂-C₆ alkenyl optionally substituted, -C₂-C₆ alkynyl optionally substituted, -C₃-C₈ cycloalkyl optionally substituted, -OC₁-C₆ alkyl optionally substituted, -OC₂-C₆ alkenyl optionally substituted, -OC₂-C₆ alkynyl optionally substituted, -OC₃-C₈ cycloalkyl optionally substituted, -NH₂, -NH(C₁-C₄ alkyl), N(C₁-C₄ alkyl)₂ or deuterium.

Preferred substituents of alkyl, alkenyl and alkynyl residues include -OH, halo, -O(C₁-C₄ alkyl), -NH₂, -NH(C₁-C₄ alkyl), -N(C₁-C₄ alkyl)₂, and deuterium.

The term "phenyl" includes phenyl residues which are optionally substituted. Preferred substituents of phenyl include -OH, halo, -C₁-C₆ alkyl optionally substituted, -C₂-C₆ alkenyl optionally substituted, -C₂-C₆ alkynyl optionally substituted, -C₃-C₈ cycloalkyl optionally substituted, -OC₁-C₆ alkyl optionally substituted, -OC₂-C₆ alkenyl optionally substituted, -OC₂-C₆ alkynyl optionally substituted, -OC₃-C₈ cycloalkyl optionally substituted, -NH₂, -NH(C₁-C₄ alkyl), N(C₁-C₄ alkyl)₂ or deuterium.

The term "heteroaryl" particularly includes 5- or 6-membered heteroaromatic residues, e.g., heteroaromatic residues comprising at least one N-, O- or S- atom in the ring which are optionally substituted. In certain embodiments, heteroaryl is furyl. Preferred substituents of heteroaryl include -OH, halo, -C₁-C₆ alkyl optionally substituted, -C₂-C₆ alkenyl optionally substituted, -C₂-C₆ alkynyl optionally substituted, -C₃-C₈ cycloalkyl optionally substituted, -OC₁-C₆ alkyl optionally substituted, -OC₂-C₆ alkenyl optionally substituted, -OC₂-C₆ alkynyl optionally substituted, -OC₃-C₈ cycloalkyl optionally substituted, -NH₂, -NH(C₁-C₄ alkyl), N(C₁-C₄ alkyl)₂ or deuterium.

The term "cycloalkyl" includes 3- to 8-membered cyclic non-aromatic and particularly saturated rings which are optionally substituted. Preferred substituents of cycloalkyl include -OH, halo, - C₁-C₆ alkyl optionally substituted, -C₂-C₆ alkenyl optionally substituted, -C₂-C₆ alkynyl optionally substituted, -C₃-C₈ cycloalkyl optionally substituted, -OC₁-C₆ alkyl optionally substituted, -OC₂-C₆ alkenyl optionally substituted, -OC₂-C₆ alkynyl optionally substituted, - OC₃-C₈ cycloalkyl optionally substituted, -NH₂, -NH(C₁-C₄ alkyl), N(C₁-C₄ alkyl)₂ or deuterium.

The term "halo" as used herein includes -F, -Cl, -Br and -I if not indicated differently.

In certain embodiments of the compounds of formula (I), R¹ is selected from -H, -OH, -CH₃ optionally substituted with -OH and/or halo, -OCH₃ optionally substituted with -OH and/or halo, phenyl or heteroaryl optionally substituted with -OH, halo, -NH₂, -NH(alkyl), N(alkyl)₂, and/or - CH₃ optionally halogenated, and -O-phenyl or -O-heteroaryl optionally substituted with -OH, halo, -NH₂, -NH(alkyl), N(alkyl)₂ and/or -CH₃ optionally halogenated.

In particular embodiments, R¹ is -CH₃, -furyl, -phenyl, -3-methylphenyl, -3-(trifluoromethyl)phenyl, -4-methylphenyl, -4-trifluoromethylphenyl, -2-chlorophenyl, -3-chlorophenyl, -4-chlorophenyl, -3,4-dimethylphenyl, -3,4-dichlorophenyl or -isopropyl. In more particular embodiments, R¹ is -CH₃.

In certain embodiments, R² is halo. In particular embodiments, R² is -Cl.

In certain embodiments, R³ is selected from -H or -CH₃, optionally substituted with halo. In particular embodiments, R³ is H.

In certain embodiments, R⁴ is H, -CH₃ optionally substituted with halo, or -R⁵. In particular embodiments, R⁴ is -R⁵. In even more particular embodiments of R⁵, X is O and/or Y is -C(O)-
In even more particular embodiments, -R⁵ is

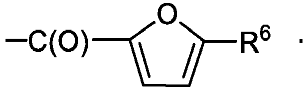

In certain embodiments, R⁶ is phenyl or heteroaryl optionally substituted. In certain embodiments, R⁶ is phenyl substituted at position 4 (p-position) relative to the position of attachment to the basic structure. In certain embodiments, R⁶ is phenyl unsubstituted or substituted with -R⁷, -OR⁸, -NR⁹R¹⁰ and/or halo, particularly Cl. In even more particular embodiments, R⁶ is phenyl substituted with -NR⁹R¹⁰,

In certain embodiments, R⁷ is -H, -C₁-C₆ alkyl or cycloalkyl, particularly -C₁-C₅ alkyl, and more particularly -CH₃ optionally substituted with halo, particularly F. In particular embodiments, R⁷ is H, -CH₃ or -CF₃.

In certain embodiments, R⁸ is H, -C₁-C₆ alkyl, particularly -C₁-C₃ alkyl, and more particularly - CH₃ optionally substituted with halo, particularly F. In particular embodiments, R⁸ is -CH₃ or - CF₃.

In certain embodiments, R⁹ and R¹⁰ are independently selected from H, -C₁-C₆ alkyl and -C₃-C₈ cycloalkyl, particularly -C₁-C₃ alkyl, and more particularly -CH₃ optionally substituted with halo. In particular embodiments, R⁹ and R¹⁰ are independently selected from -H and -CH₃. In even more particular embodiments, R⁹ and R¹⁰ are -CH₃.

A particularly preferred compound of formula (I) is 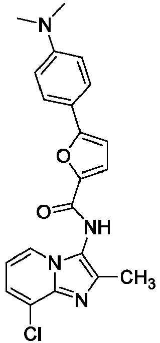 or a salt or solvate thereof.

The present invention also encompasses salts of the compounds of formula I, in particular pharmaceutically acceptable salts. The compounds of the present invention are capable of forming acid and/or base salts by means of the presence of basic and/or acidic groups. Acid addition salts may be formed with inorganic acids and organic acids and include, but are not limited to, acetate, benzoate, hydrobromide, hydrochloride, citrate, lactate, phosphate, tosylate and trifluoroacetate salts. Base addition salts can be formed with inorganic bases and organic bases and include, but are not limited to, ammonium, sodium, potassium, calcium, magnesium salts or salts derived from primary, secondary, and tertiary amines.

Also included are solvates, e.g. hydrates, of the compounds of formula (I) and solvates of their salts.

A further aspect of the present invention relates to the use of a compound of formula (I) or a salt or solvate thereof in medicine, e.g., in human or veterinary medicine. The compounds of formula (I) may be administered to a subject, e.g., a human subject, in need thereof for preventing a disease (particularly to a subject being at risk of acquiring a disease) or for treating a disease (particularly to a subject already suffering from a disease).

A further aspect of the present invention relates to a pharmaceutical composition comprising the compound of formula (I) or a salt or solvate thereof as an active agent and a pharmaceutically acceptable carrier. The composition may be a solid dosage form, e.g. a tablet, a capsule etc. or a liquid dosage form, e.g. a solution, emulsion, suspension, or the like. It is administered in any suitable way, e.g. orally, parenterally, by inhalation, by ocular application, by intramuscular application, by nasal application and/or by transdermal application. The carrier may be any suitable pharmaceutical carrier as known in the art. The compound of Formula I is administered in a therapeutically effective dose which may be determined by the skilled practitioner based on the subject and the disorder to be treated.

A further aspect of the present invention relates to the use of the compound of formula (I) or a salt or solvate thereof in the prevention and/or treatment of a disease caused by and/or associated with insufficient Nurr1 activity. The term "insufficient Nurr1 activity" is understood that the disease may be prevented, ameliorated and/or cured by increasing the Nurr1 activity in a subject in need thereof, e.g., a human subject.

A further aspect of the present invention relates to the use of the compound of formula (I) or a salt or solvate thereof in the prevention and/or treatment of a degenerative disease, an inflammatory disease and/or a hyperproliferative disease such as cancer, e.g., a degenerative disease, an inflammatory disease, and/or a hyperproliferative disease such as cancer caused by and/or associated with insufficient Nurr1 activity.

A further aspect of the present invention relates to the use of the compound of formula (I) or a salt or solvate thereof in the prevention and/or treatment of a neurodegenerative disease caused by and/or associated with insufficient Nurr1 activity.

A further aspect of the present invention relates to the use of the compound of formula (I) or a salt or solvate thereof in the prevention and/or treatment of an ocular disease caused by and/or associated with insufficient Nurr1 activity.

A further aspect of the present invention relates to the use of the compound of formula (I) or a salt or solvate thereof in the prevention and/or treatment of a gastro-intestinal disease caused by and/or associated with insufficient Nurr1 activity.

A further aspect of the present invention relates to the use of the compound of formula (I) or a salt or solvate thereof in the prevention and/or treatment of cancer caused by and/or associated with insufficient Nurr1 activity.

A further aspect of the present invention relates to the use of the compound of formula (I) or a salt or solvate thereof in in the prevention and/or treatment of a disease selected from Morbus Alzheimer, Morbus Parkinson, dementia, multiple sclerosis, progressive supranuclear palsy (PSP), ischemic stroke, schizophrenia, depression, ADHD, circadian rhythm disorder, rheumatoid arthritis, lupus erythematosus, or inflammatory bowel disease, or macular degeneration including age-related macular degeneration.

A further aspect relates to method of preventing and/or treating a degenerative or inflammatory disease comprising administering to a subject in need thereof an effective amount of a compound of formula (I).

A compound of formula (I) may be administered as a monotherapy or as a combination therapy, i.e., in combination with at least one further medicament different from a compound of formula (I), particularly at least one further medicament suitable for the treatment of a degenerative and/or inflammatory disease as described above. More particularly a compound of formula (I) may be used in combination therapy with levodopa, benserazide, opicapone, entacapone, tolcapone, bromocriptine, pergolide, pramipexole, ropinirole, piribedil, cabergoline, apomorphine, safinamide, selegiline, rasagiline, lisuride, amantadine, donzepezil, rivastigmine, galantamine, memantine, glucocorticoids, glatiramer acetate, interferon beta, teriflunomide, dimethyl fumarate, fingolimod, ozanimod, ofatumumab, alemtuzumab, ocrelizumab, azathioprine, or methotrexate.

Further, the compounds of formula (I) are also useful for non-medical applications, e.g., in the fields of basic research, diagnostics and/or drug screening.

The compounds of formula (I) may be prepared by synthesis procedures as described herein.

Compounds **7, 8, 12** and **14-24** were prepared by a Groebke-Blackburn reaction according to Scheme 1. 3-Chloropyridin-2-amine (**39**) and 4-chloropyridin-2-amine (**40**) were cyclized with the aldehydes **41-53** and 1,1,3,3-tetramethylbutylisocyanide followed by acid-mediated cleavage to **7**, **8** and **14-24** using 4 N HCl in dioxane or TFA/CH₂Cl₂ (1:1). 1,1,3,3-Tetramethylbutylisocyanide **59** was commercially available. 3-Amino-8-chloroimidazo[1,2-a]pyridine **12** was prepared by nitration of 8-chloroimidazo[1,2-a]pyridine **60** followed by reduction with iron. 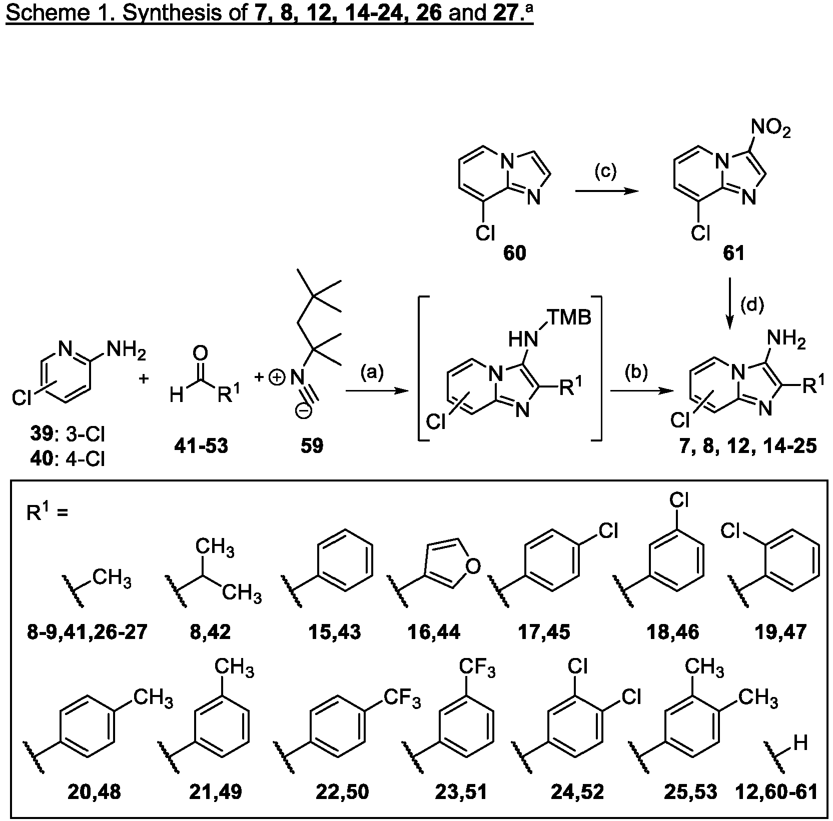 a: Reagents & Conditions: (a) AcOH, MeOH, rt, 24-48 h; (b) TFA/DCM, rt, 30 min to overnight, 3-30% over two steps; (c) nitric acid, sulfuric acid, 0°C - rt, 1 h, 93%; (d) iron, ammonium chloride, MeOH/water, reflux, overnight, 57%.

Compounds **26-36** were obtained by amide coupling of 3-amino-8-chloro-2-methylimidazo[1,2-a]pyridine (**8**) and the carboxylic acids **68-77** using HATU or thionyl chloride (Scheme 2). The carboxylic acids **68** and **69** were prepared by Suzuki coupling of aryl bromide 63 with the boronates **64**, **65** to **66**, **67** followed by alkaline ester hydrolysis. **70-77** were commercially available. 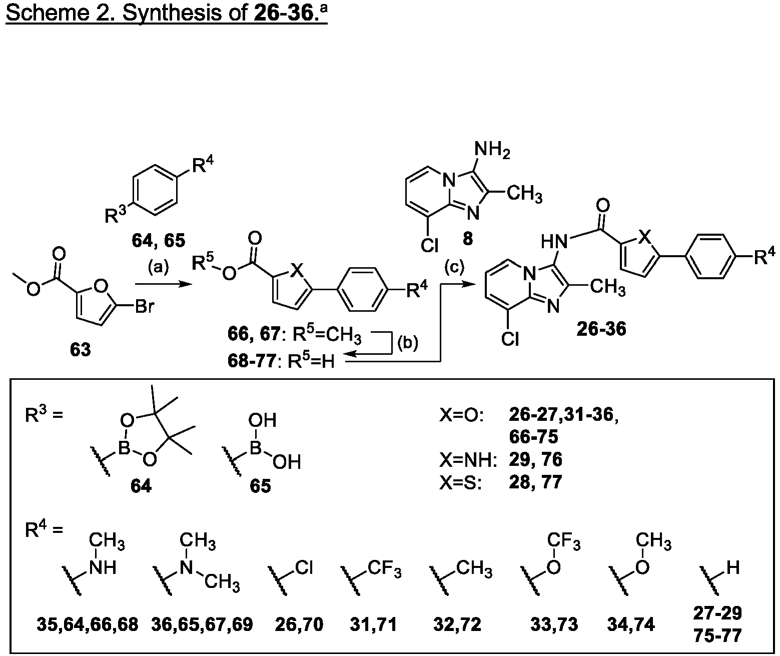 a: Reagents & Conditions: (a) XPhos Pd G2, K₃PO₄, water/dioxane, reflux, overnight, 86-97%; (b) LiOH, water/THF, rt, overnight, 67-73%; (c) HATU, DIPEA, DMF, rt, overnight, 33-100% or SOCl₂, CHCl₃ reflux, 3 h, 11%.

In the following, the present invention shall be explained in more detail by the Figures and Examples.

### FIGURE LEGENDS

**Figure 1****.** Orthogonal validation of **8** (a), **24** (b) and **26** (c) as direct Nurr1 ligands by isothermal titration calorimetry (ITC). The fittings of the heat of binding are shown and the isotherms at 25°C are shown as insets.

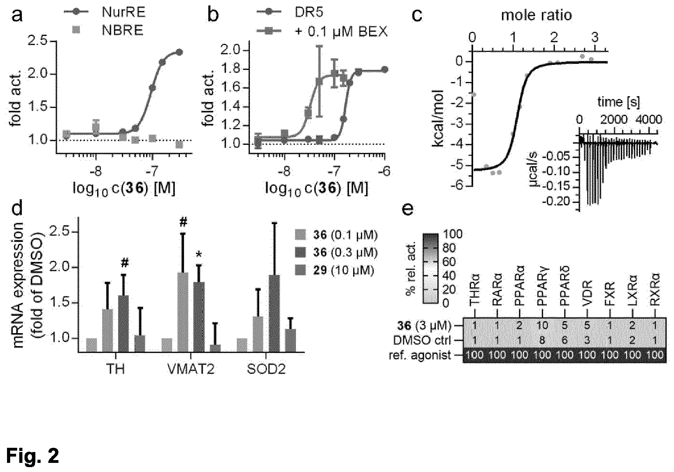

**Figure 2****.** Orthogonal validation and profiling of **36.** (a) **36** robustly activated the human full-length Nurr1 homodimer (NurRE) but not the monomer (NBRE). Data are the mean±S.E.M. fold activation vs. DMSO ctrl; n≥3. (b) **36** activated the Nurr1-RXR heterodimer (DR5) and synergized with bexarotene (0.1 µM). Data are the mean±S.E.M. fold activation vs. DMSO ctrl or vs. 0.1 µM BEX; nz3. (c) ITC confirmed binding of **36** to the Nurr1 LBD (K_{d} 0.17 µM, n=1.0).

The fitting of the heat of binding is shown and the isotherm at 25°C is shown as inset. (d) Effects of **36** on the expression of the Nurr1-regulated tyrosine hydroxylase (TH), vesicular amino acid transporter 2 (VMAT2) and superoxide dismutase 2 (SOD2) in astrocytes (T98G). **29** had no effect. Data are the mean±S.E.M. fold mRNA induction vs. DMSO control; n=3; ^{#} p<0.1, * p<0.05 (t-test vs. DMSO ctrl). (e) Selectivity screening of **36** revealed no off-target activity on nuclear receptors. Heatmap shows the mean relative activation compared to reference ligands (listed in methods section); n=3.

### EXAMPLES

### 1. Experimental

### 1.1 Chemistry

*General.* All chemicals were of reagent grade, purchased from commercial sources (e.g., Sigma-Aldrich, TCI, BLDpharm) and used without further purification unless otherwise specified. All reactions were conducted under nitrogen or argon atmosphere and in absolute solvents purchased from Sigma-Aldrich. Other solvents, especially for work-up procedures, were of reagent grade or purified by distillation (iso-hexane, cyclohexane, ethyl acetate, EtOH). Reactions were monitored by thin layer chromatography (TLC) on TLC Silica gel 60 F254 coated aluminum sheets by Merck and visualized under ultraviolet light (254 nm) or by using ninhydrin or Ehrlichs reagent stains. Purification by column chromatography was performed on a puriFlash^{®} XS520Plus system (Advion, Ithaca, NY, USA) using high performance spherical silica columns (SIHP, 50 µM) by Interchim and a gradient of iso-hexane or cyclohexane to ethyl acetate, Reversed-phase column chromatography was performed on a puriFlash^{®} 5.250 system (Advion) using C18HP columns (SIHP, 15 µM) by Interchim and a gradient of H₂O with 10% MeCN to 100% MeCN (HPLC gradient grade). Mass spectra were obtained on a puriFlash^{®}-CMS system (Advion) using atmospheric pressure chemical ionization (APCI). HRMS were obtained with a Thermo Finnigan LTQ FT instrument for electron impact ionization (EI) or electrospray ionization (ESI). NMR spectra were recorded on Bruker Avance III HD 400 MHz or 500 MHz spectrometers equipped with a CryoProbeTM Prodigy broadband probe (Bruker). Chemical shifts are reported in δ values (ppm), coupling constants (*J*) in hertz (Hz). The purity of the compounds was determined by ¹H NMR (qHNMR) according to the method described by Pauli et al.³⁸ with internal calibration. To ensure accurate determination of peak area ratio, the qHNMR measurements were conducted under conditions allowing for complete relaxation. Ethyl 4-(dimethylamino)benzoate (LOT#BCCC6657, purity 99.63%), dimethyl terephthalate (LOT#BCBT9974, purity 99.95%) and maleic acid (LOT#BCBM8127V, purity 99.94%) were used as internal standards in MeOD-d₄, DMSO-d₆, or acetone-d₆. All compounds for biological testing had a purity >95% according to quantitative ¹H NMR (qHNMR).

*General procedure A for Groebke-Blackburn-Bienaymé reaction and hydrolysis.* 2-Amino-3-chloropyridine (**39**, 1.0 eq.) or 2-amino-4-chloropyridine (**40**, 1.0 eq.), aldehyde (**41-53,** 1.0-1.1 eq.) and glacial acetic acid (1.5 eq.) were dissolved in dry methanol (0.7 M) under nitrogen atmosphere. The mixture was stirred for 30 - 45 min. at room temperature (rt) for imine formation. 1,1,3,3-Tetramethylbutylisocyanid (**59**, 1.5 eq.) was subsequently added, and the mixture was stirred at rt for 17 - 48 h. When TLC monitoring indicated completion, the isocyanide was quenched by addition of 2 N HCl (2 mL) and further stirring for 30 min. 2 N NaOH and ethyl acetate (10 mL) were then added, and the phases were separated. The aqueous layer was extracted with ethyl acetate (3x). The combined organic layers were dried over MgSO₄ and the solvent was evaporated under reduced pressure. The crude product was dissolved in a mixture of CH₂Cl₂ and trifluoroacetic acid (10 mL, 1:1) or 4N HCl in dioxane (10 mL) and the mixture was stirred for 30-60 min. at rt. When TLC monitoring indicated completion, 2 N NaOH was added, phases were separated, and the aqueous layer was extracted with ethyl acetate (3x). The combined organic layers were dried over MgSO₄ and the solvents were evaporated under reduced pressure. The crude product was purified by flash chromatography using a gradient of iso-hexane or cyclohexane / ethyl acetate as mobile phase, and potentially by reverse phase chromatography using a gradient of H₂O with 10% MeCN to 100% MeCN (HPLC gradient grade).

*General procedure B for amide coupling with HATU.* The corresponding carboxylic acid (**68-76**, 1.2 eq.) and 1-[bis(dimethylamino)methylene]-1*H*-1,2,3-triazolo[4,5-b]pyridinium 3-oxide hexafluorophosphate (HATU, 1.2 eq.) were dissolved in DMF (0.13 M). Ethyldiisopropylamine (DIPEA, 0.13 M, 1.2 eq) was added and the mixture was stirred at rt for 40 min. The corresponding 3-aminoimidazo[1,2-a]pyridine (**8**, 1.0 eq.) was dissolved in DMF (0.11 M) and added to the activated carboxylic acid. The mixture was stirred at rt overnight. When TLC monitoring indicated completion, the solvent was removed under reduced pressure, the residue was dissolved in ethyl acetate and treated with 5% HCl (0.13 M). Phases were separated and the aqueous layer was extracted with ethyl acetate (3x). The combined organic layers were washed with 1 N NaOH and dried over MgSO₄. The solvents were evaporated under reduced pressure and the crude product was purified by flash chromatography using a gradient of *iso*-hexane / ethyl acetate as mobile phase, and potentially by reverse phase chromatography using a gradient of H₂O with 10% MeCN to 100% MeCN (HPLC gradient grade).

*8-Chloro-2-methylimidazo[1,2-a]pyridine-3-amine* (**8**). Preparation according to general procedure A, using 2-amino-3-chloropyridine (**39**, 821 mg, 6.39 mmol, 1.00 eq) and acetaldehyde (**41**, 39 µL, 7.03 mmol, 1.10 eq). **8** was obtained as a colorless solid (344 mg, 30%). ¹H-NMR (400 MHz, acetone-*d*₆): δ = 8.05 (dd, *J* = 6.8, 1.1 Hz, 1H), 7.11 (dd, *J* = 7.3, 1.0 Hz, 1H), 6.76 (t, *J* = 7.0 Hz, 1H), 4.17 (s, 2H), 2.33 (s, 3H).¹³C-NMR (101 MHz, acetone-*d*₆): δ = 136.11, 130.11, 126.33, 121.50, 121.00, 119.83, 110.01, 11.89. MS (APCI+): *m*/*z* 181.9 ([M+H]⁺). HRMS (EI+): *m*/*z* calculated 181.0407 for C₈H₈ClN₃, found 181.0400 ([M+H]⁺). qHNMR (400 MHz, acetone-*d*₆, ethyl-4(dimethylamino)benzoate as reference): purity = 97.5%.

*8-Chloro-2-(3,4-dichlorophenyl)imidazo[1,2-a]pyridine-3-amine* (**24**). Preparation according to general procedure A, using 2-amino-4-chlorpyridine (**39**, 1.29 g, 10.0 mmol, 1.00 eq) and 3,4-dichlorobenzaldehyde (**52**, 1.93 g, 11.0 mmol, 1.01 eq). **24** was obtained as a yellow solid (197 mg, 6%). ¹H-NMR (400 MHz, acetone-*d*₆): δ = 8.41 - 8.35 (m, 1H), 8.30 - 8.23 (m, 1H), 8.20 - 8.12 (m, 1H), 7.63 - 7.56 (m, 1H), 7.27 (dd, *J* = 7.3, 1.1 Hz, 1H), 6.88 (t, *J* = 7.1 Hz, 1H), 4.83 (s, 2H).¹³C-NMR (101 MHz, MeOD-*d*₄): δ = 137.16, 134.44, 132.08, 130.14, 130.03, 128.55, 127.99, 127.52, 126.32, 122.59, 121.74, 121.32, 111.34. MS (APCI+): *m*/*z* 311.9 ([M+H]⁺). HRMS (EI+): *m*/*z* calculated 310.9784 for C₁₃H₈Cl₃N₃, found 310.9778 ([M]•⁺). qHNMR (400 MHz, acetone-*d*₆, ethyl-4-(dimethylamino)benzoate as reference) purity = 95.7%.

*N-(8-Chloro-2-methylimidazo[1,2-a]pyridin-3-yl)-5-(4-(dimethylamino)phenyl)furan-2-carboxamide* (**36**). Preparation according to general procedure B using 8-chloro-2-methylimidazo[1,2-a]pyridine-3-amine (**8**, 50 mg, 0.28 mmol, 1.00 eq) and 5-(4-(dimethylamino)phenyl)furan-2-carboxylic acid (**69**, 76 mg, 0.33 mmol, 1.20 eq). **36** was obtained as a yellow solid (69 mg, 64%). ¹H-NMR (400 MHz, acetone-*d*₆): δ = 9.68 (s, 1H), 8.11 (d, *J* = 1.0 Hz, 1H), 7.77 (d, *J* = 8.5 Hz, 2H), 7.40 - 7.27 (m, 2H), 6.94 - 6.73 (m, 4H), 3.00 (s, 6H), 2.36 (s, 3H). ¹³C-NMR (101 MHz, acetone-*d*₆): δ = 157.70, 157.19, 151.01, 145.08, 139.07, 138.69, 125.87, 122.80, 122.61, 121.82, 117.98, 117.58, 117.02, 111.99, 110.99, 104.28, 39.38, 12.36. MS (APCI+): *m*/*z* 394.7 ([M+H]⁺). HRMS (EI+): *m*/*z* calculated 394.1197 for C₂₁H₁₉ClN₄O₂, found 394.1194 ([M]•⁺). qHNMR (400 MHz, acetone-*d*₆, maleic acid as reference) purity = 95.1%.

### 1.2 In vitro Characterization

*Hybrid reporter gene assays.* Nurr1 modulation was determined in a Gal4 hybrid reporter gene assay in HEK293T cells (German Collection of Microorganisms and Cell Culture GmbH, DSMZ) using pFR-Luc (Stratagene, La Jolla, CA, USA; reporter), pRL-SV40 (Promega, Madison, WI, USA; internal control) and pFA-CMV-hNurr1-LBD¹⁴, coding for the hinge region and ligand binding domain of the canonical isoform of human Nurr1. Cells were cultured in Dulbecco's modified Eagle's medium (DMEM), high glucose supplemented with 10% fetal calf serum (FCS), sodium pyruvate (1 mM), penicillin (100 U/mL), and streptomycin (100 µg/mL) at 37 °C and 5% CO₂ and seeded in 96-well plates (3×10⁴ cells/well). After 24 h, medium was changed to Opti-MEM without supplements and cells were transiently transfected using Lipofectamine LTX reagent (Invitrogen, Carlsbad, CA, USA) according to the manufacturer's protocol. Five hours after transfection, cells were incubated with the test compounds in Opti-MEM supplemented with penicillin (100 U/mL), streptomycin (100 µg/mL) and 0.1% DMSO for 16 h before luciferase activity was measured using the Dual-Glo Luciferase Assay System (Promega) according to the manufacturer's protocol on a Tecan Spark luminometer (Tecan Deutschland GmbH, Crailsheim, Germany). Firefly luminescence was divided by Renilla luminescence and multiplied by 1000 resulting in relative light units (RLU) to normalize for transfection efficiency and cell growth. Fold activation was obtained by dividing the mean RLU of test compound by the mean RLU of the untreated control. All samples were tested in at least three biologically independent experiments in duplicates. For dose-response curve fitting and calculation of EC₅₀ values, the equation "[Agonist] vs. response -- Variable slope (four parameters)" was used in GraphPad Prism (version 7.00, GraphPad Software, La Jolla, CA, USA). Selectivity profiling was performed with identical procedures using pFA-CMV-Nur77-LBD¹⁴, pFA-CMV-NOR-1-LBD¹⁴, pFA-CMV-THRα-LBD³⁹, pFA-CMV-RARα-LBD⁴⁰, pFA-CMV-PPARα-LBD⁴¹, pFA-CMV-PPARγ-LBD⁴¹, pFA-CMV-PPARδ-LBD⁴¹, pFA-CMV-LXRα-LBD³⁷, pFA-CMV-FXR-LBD⁴² and pFA-CMV-hRXRα-LBD⁴³.

*Full-length Nurr1 reporter gene assays.* Activation of full length human Nurr1 was studied in transiently transfected HEK293T cells using the reporter plasmids pFR-Luc-NBRE¹⁴, pFR-Luc-POMC¹⁴ or pFR-Luc-DR5¹⁴ each containing one copy of the respective human Nurr1 response element NBRE Nl3, NurRE, or DR5. The full length human nuclear receptor Nurr1 (pcDNA3.1-hNurr1-NE; Addgene plasmid #102363) and, for DR5, RXRα (pSG5-hRXR)⁴⁴ were overexpressed. pRL-SV40 (Promega) was used for normalization of transfection efficacy and to observe test compound toxicity. Cells were cultured in Dulbecco's modified Eagle's medium (DMEM), high glucose supplemented with 10% fetal calf serum (FCS), sodium pyruvate (1 mM), penicillin (100 U/mL), and streptomycin (100 µg/mL) at 37 °C and 5 % CO₂ and seeded in 96-well plates (3×10⁴ cells/well). After 24 h, medium was changed to Opti-MEM without supplements and cells were transiently transfected using Lipofectamine LTX reagent (invitrogen) according to the manufacturer's protocol. Five hours after transfection, cells were incubated with the test compounds in Opti-MEM supplemented with penicillin (100 U/mL), streptomycin (100 µg/mL) and 0.1% DMSO for 16 h before luciferase activity was measured using the Dual-Glo Luciferase Assay System (Promega) according to the manufacturer's protocol on a Tecan Spark luminometer (Tecan Deutschland GmbH). Firefly luminescence was divided by Renilla luminescence and multiplied by 1000 resulting in relative light units (RLU) to normalize for transfection efficiency and cell growth. Fold activation was obtained by dividing the mean RLU of test compound by the mean RLU of the untreated control. All samples were tested in at least three biologically independent experiments in duplicates. For dose-response curve fitting and calculation of EC₅₀ values, the equation "[Agonist] vs. response -- Variable slope (four parameters)" was used in GraphPad Prism (version 7.00, GraphPad Software).

*Isothermal Titration Calorimetry (ITC).* ITC experiments were conducted on an Affinity ITC instrument (TA Instruments, New Castle, DE) at 25°C with a stirring rate of 75 rpm. Nurr1 LBD protein (10-30 µM, expressed as described previously²¹) in buffer (20 mM Tris pH 7.5, 100 mM NaCl, 5% glycerol) containing 1-4% DMSO was titrated with the test compounds (60-150 µM in the same buffer containing 1-4% DMSO) in 26 injections (1x 1 µL, 25x 3-4 µL) with an injection interval of 150 s. As control experiments, the test compounds were titrated to the buffer, and the buffer was titrated to the Nurr1 LBD protein under otherwise identical conditions. Results were analyzed using NanoAnalyze software (version 3.11.0, TA Instruments, New Castle, DE) with independent binding models.

*Evaluation of Nurr1-regulated VMAT2 expression in T98G cells.* T98G (ATCC CRL-1690^{™}) were grown in DMEM, high glucose supplemented with 10% FCS, sodium pyruvate (1 mM), penicillin (100 U/mL), and streptomycin (100 µg/mL) at 37°C and 5% CO₂ and seeded at a density of 250,000 cells per well in 12-well plates. After 24 h, medium was changed to DMEM, high glucose supplemented with 0.2% fetal calf serum (FCS), penicillin (100 U/mL) and streptomycin (100 µg/mL) and the cells were incubated for another 24 h before stimulation with the test compounds (**36** (0.3 µM), **29** (10 µM)) solubilized with 0.1% DMSO or with 0.1% DMSO as negative control. After 16 h of incubation the medium was removed, cells were washed with phosphate buffered saline (PBS) and after full aspiration of residual liquids immediately frozen at -80 °C until further processing. Total RNA was isolated using the E.Z.N.A.^{®} Total RNA Kit I (Omega Bio-tek, Norcross, USA) following the manufacturer's instructions. RNA concentration and purity was assessed using a NanoDrop^{™} One UV/VIS spectrophotometer (Thermo Fisher Scientific, Waltham, USA) at 260/280 nm. Right before reverse transcription (RT), RNA was linearized at 65 °C for 10 min and then immediately incubated on ice for at least 1 min. Reverse transcription was performed using 2 µg total RNA, 20 U Recombinant RNasin^{®} Ribonuclease Inhibitor (Promega, Mannheim, Germany), 100 U SuperScript ^{®} IV Reverse Transcriptase including 5x First Strand Buffer and 0.1 M dithiothreitol (Thermo Fisher Scientific, Waltham, USA), 3.75 ng linear acrylamide, 625 ng random hexamere primers (#11277081001, Merck, Darmstadt, Germany) and 11.25 nmol deoxynucleoside triphosphate mix (2.8 nmol each ATP, TTP, CTP, GTP; #R0186, Thermo Fisher Scientific, Waltham, USA) at a volume of 22.45 µL at 50 °C for 10 min and 80 °C for 10 min using a Thermal cycler XT⁹⁶ (VWR International, Darmstadt, Germany). Quantitative polymerase chain reaction (qPCR) was conducted using an Applied Biosystems^{™} QuantStudio 1 (Waltham, USA) and a SYBR green based detection method. Appropriately diluted cDNA was added to 6 pmol of forward and reverse primer, respectively, 0.8 U Taq DNA Polymerase (#M0267, New England Biolabs, Ipswich, USA), 40 ppm SYBR^{®} Green I (#S9430, Sigma Aldrich, St. Louis, USA), 15 nmol deoxynucleoside triphosphate mix (as indicated above), 60 nmol MgCl₂, 4 µg bovine serum albumin (#B14, Thermo Fisher Scientific, Waltham, USA), 20% BioStab PCR Optimizer II (#53833, Merck, Darmstadt, Germany), and 10% Taq buffer without detergents (#B55, Thermo Fisher Scientific, Waltham, USA) topped up at a final volume of 20 µL with ddHzO. Samples underwent 40 cycles of 15 s denaturation at 95 °C, 15 s of primer annealing at 59.4 or 62.4 °C (depending on the primer), and 20 s of elongation at 68 °C. PCR product specificity was evaluated using a melting curve analysis ranging from 65 to 95 °C. VMAT2 mRNA expression was normalized to GAPDH mRNA expression per each sample using the ΔCt-method. The following primers for the human genes were used. hVMAT2 (SLC18A2): 5'-GCT ATG CCT TCC TGC TGA TTG C-3' (fw) and 5'-CCAAGG CGATTC CCATGA CGT T-3' (rev); hGAPDH: 5'-AGG TCG GAG TCAACG GAT TT-3' (fw) and 5'-TTC CCG TTC TCA GCC TTG AC-3' (rev).

*Determination* of *aqueous solubility.* The aqueous solubility of **29** and **36** was assessed by mixing 1 mg of each test compound with an appropriate volume of water for a theoretical concentration of 4 mM to obtain an oversaturated mixture. The mixture was agitated in a VWR Thermal Shake lite (VWR International GmbH, Darmstadt, Germany) for 24 h at 600 rpm and constant temperature of 25°C. The supersaturated mixtures were subsequently centrifuged at 23.300 rpm for 15 min (25 °C). Part of the supernatant was taken off for quantification by UV absorbance at 312 nm with external calibration. The external calibration samples contained 1% DMSO and the test samples were spiked with DMSO to 1% concentration right before the measurement. Absorbance was measured with a Tecan Spark luminometer (Tecan Deutschland GmbH, Crailsheim, Germany). The solubility test was repeated in three independent experiments,

### 2. Results

We commenced the development of AQ-derived Nurr1 modulators by probing replacement of the quinoline scaffold of the optimized AQ fragment 4 by alternative heterocycles (Table 1). The corresponding quinazoline 5 was inactive and 7-chloro-2-methyl-1H-indol-3-amine (**6**) was not stable. 4-Chloro-2-methyl-benzimidazole (7) also failed to activate Nurr1 suggesting that the amino substituent was required and could not be replaced by the ring NH.

**Table 1. Optimization of the chloroquinoline fragment**

| ID | structure | EC₅₀(Nurr1) (max. activation)^{a} |
|---|---|---|
| **4** | 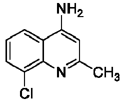 | 17±6 µM (1.7±0.1-fold) |
| **5** | 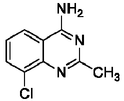 | inactive (100 µM) |
| **6** | 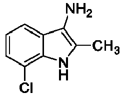 | unstable |
| **7** | 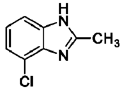 | inactive (100 µM) |
| **8** | 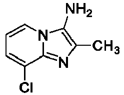 | 7±1 µM (2.0±0.1-fold) |
| **9** | 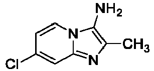 | inactive (100 µM) |
| **10** | 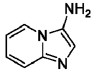 | inactive (100 µM) |
| **11** | 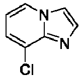 | inactive (100 µM) |
| **12** | 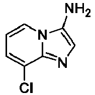 | inactive (100 µM) |
| **13** | 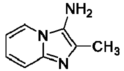 | inactive (100 µM) |

| | | |
|---|---|---|
| ^{a} Nurr1 modulation was determined in a Gal4-Nurr1 hybrid reporter gene assay. Max. activation refers to the maximum effect vs. 0.1% DMSO control. Data are the mean±SD; nz3. | | |

The alternative imidazo[1,2-*a*]pyridine scaffold (**8**), in contrast, retained Nurr1 agonism and achieved a notable improvement in potency and efficacy compared to **4.** The smaller skeleton was not compatible with the original regiochemistry of the chloro substituent (**9**) and systematic deconstruction of **8** highlighted importance of all substituents (**10-13**). With single-digit micromolar potency and affinity (K_{d} 2.7 µM, Figure 1a), the imidazo[1,2-*a*]pyridine **8** hence emerged as an improved fragment-like Nurr1 agonist which was also evident from enhanced ligand efficiency (LE), lipophilic ligand efficiency (LLE), and size-independent ligand efficiency (SILE) compared toAQ (**1**) and the lead fragment **4** (Table 2).

**Table 2. Ligand efficiency metrics of 1, 4 and 8.^{a}**

| | **1** | **4** | **8** |
|---|---|---|---|
| ligand efficiency (LE) | 0.26 | 0.50 | 0.59 |
| lipophilic ligand efficiency (LLE) | - 0.48 | 1.99 | 3.28 |
| size-independent ligand eff. (SILE) | 1.79 | 2.21 | 2.45 |

| | | | |
|---|---|---|---|
| ^{a} Efficiency metrics were computed according to²⁹. | | | |

Aiming to enhance potency by fragment growing, we next evaluated a potential optimization of the 2-methyl substituent of **8** (Table 3). Extension in this region caused a general reduction in Nurr1 activation efficacy. In a rough exploration of aliphatic (**14**) and aromatic (**15**, **16**) extensions, only the phenyl substituent (**15**) retained sufficient efficacy and provided a slight improvement over **8** in terms of potency (EC₅₀ 4 µM). Chloro substituents in 4- (**17**) and 3- (**18**) positions of the phenyl motif enhanced activation efficacy but diminished potency. 2-Chloro substitution (**19**) disrupted activity on Nurr1. Replacement of the chloro substituents (**17**, **18**) by a methyl group was tolerated in 4-position (**20**) but not in 3-position (**21**), and trifluoromethyl substituents (**22**, **23**) also caused a marked drop in efficacy. Double 3,4-chloro substitution (**24**), in contrast, was additive and resulted in enhanced potency (EC₅₀ 0.4 µM) and efficacy (1.8-fold activation). The corresponding dimethyl analogue **25** was less active.

Although structural extension of the 2-methyl substituent in **8** provided no major improvement in Nurr1 agonist activity, the 3,4-dichlorophenyl derivative **24** was favored and achieved a 7-fold increase in potency over **8**. which was in line with results from ITC showing enhanced affinity of **24** (K_{d} 0.7 µM, Figure 1b). The 2-(3,4-dichlorophenyl) substituent of **24** may thus be a valuable potency driving extension in fused derivatives.

**Table 3. Extension of the imidazo[1,2-a]pyridine 8**

| ID | R1= | 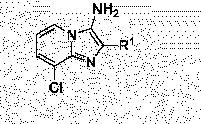 | EC₅₀(Nurr1) |
|---|---|---|---|
| | | | (max. activation)^{a} |
| **8** | 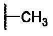 | | 7±1 µM (2.0±0.1-fold) |
| **14** | 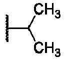 | | inactive (100 µM) < 1.2-fold act. |
| **15** | 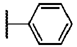 | | 412 µM (1.4±0.1-fold) |
| **16** | 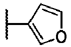 | | inactive (100 µM) < 1.2-fold act. |
| **17** | 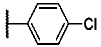 | | 15±3 µM (1.7±0.2-fold) |
| **18** | 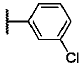 | | 9±3 µM (1.7±0.1-fold) |
| **19** | 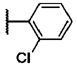 | | inactive (100 µM) |
| **20** | 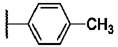 | | 12±2 µM (2.0±0.2-fold) |
| **21** | 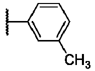 | | < 1.2-fold act. |
| **22** | 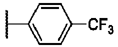 | | < 1.2-fold act. |
| **23** | 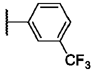 | | < 1.2-fold act. |
| **24** | 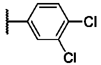 | | 0.4±0.2 µM (1.8±0.1-fold) |
| **25** | 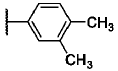 | | 14±2 µM (1.6±0.1-fold) |

| | | | |
|---|---|---|---|
| ^{a} Nurr1 modulation was determined in a Gal4-Nurr1 hybrid reporter gene assay. Max. activation refers to the maximum effect vs. 0.1% DMSO control. Data are the mean±SD; nz3. | | | |

The optimized fragment Nurr1 agonist **8** obtained by fragment hopping from the chloroquinoline motif of AQ also appeared suitable for fragment growing by fusion with N-substituents. Our previous studies³⁰ have revealed a 5-(4-chlorophenyl)furan-2-carboxamide residue as alternative motif to replace the aminophenol of AQ (**1**). Transfer of this SAR knowledge to the new imidazo[1,2-a]pyridine scaffold of **8** in the fused 5-(4-chlorophenyl)furan-2-carboxamide derivative **26** provided a notable improvement in Nurr1 agonist potency (Table 4). Simplification of **26** by removal of the chloro substituent (**27**) was tolerated but among alternative central aromatic systems only thiophen (**28**) retained Nurr1 agonism while replacement of furan (**27**) by pyrrole (**29**) or benzene (**30**) disrupted activity thus suggesting **26** as lead for further optimization.

**Table 4. Fusion of 8 with N-substituents**

| | 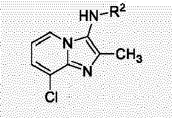 | EC₅₀(Nurr1) |
|---|---|---|
| ID | R²= | (max.activation)^{a} |
| **8** | 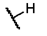 | 7±1 µM (2.0±0.1-fold) |
| **26** | 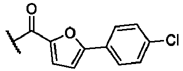 | 1.6±0.5 µM (1.8±0.1-fold) |
| **27** | 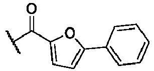 | 4±1 µM (2.1±0.1-fold) |
| **28** | 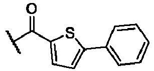 | 3.2±0.4 µM (2.1±0.1-fold) |
| **29** | 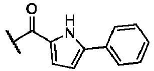 | inactive (10 µM) |
| **30** | 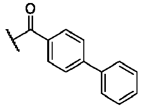 | inactive (10 µM) |

| | | |
|---|---|---|
| ^{a} Nurr1 modulation was determined in a Gal4-Nurr1 hybrid reporter gene assay. Max. activation refers to the maximum effect vs. 0.1% DMSO control. Data are the mean±SD; n≥3. | | |

Favorable Nurr1 agonist potency and improved binding affinity (K_{d} 0.7 µM, Figure 1c) highlighted **26** as attractive Nurr1 agonist scaffold derived from AQ (**1**) but further improvement of potency was required. The imidazo[1,2-*a*]pyridine and phenylfuran-2-carboxamide skeletons already appeared highly favored according to the SAR evaluation, but optimization potential seemed to rest in the 4-chloro substituent as its removal (**27**) hardly diminished potency. This suggested that space to accommodate substituents was available in this region but alternative motifs to replace the chlorine atom were needed. Hence, we focused our attention on varying the 4-substituent of the phenylfuran-2-carboxamide residue (Table 5).

The 4-trifluoromethyl derivative **31** exhibited similar activity as **26** while the corresponding 4-methyl analogue **32** was substantially more potent. A similar trend was observed for the 4-trifluoromethoxy (**33**) and 4-methoxy (**34**) pair, which indicated potential relevance of inductive effects. Like 4-methyl (**32**) and 4-methoxy (**34**), a 4-methylamino substituent (**35**) was highly favored and enhanced Nurr1 agonist potency to a sub-micromolar range. A 4-dimethylamino group (**36**) provided further improvement to a double-digit nanomolar EC₅₀ value.

**Table 5. Optimization of the fused derivative 26**

| | 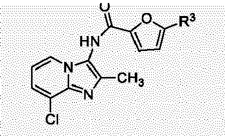 | EC₅₀(Nurr1) |
|---|---|---|
| ID | R3= | (max.activation)^{a} |
| **27** | 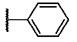 | 4±1 µM (2.1±0.1-fold) |
| **26** | 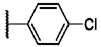 | 1.6±0.5 µM (1.8t0.1-fold) |
| **31** | 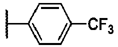 | 1.1±0.3 µM (1.8t0.1-fold) |
| **32** | 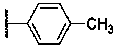 | 0.13±0.02 µM (2.0±0.2-fold) |
| **33** | 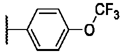 | 0.8±0.2 µM (2.010.1-fold) |
| **34** | 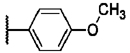 | 0.16±0.06 µM (1.7±0.1-fold) |
| **35** | 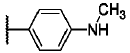 | 0.30±0.02 µM (1.8t0.1-fold) |
| **36** | 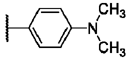 | 0.090±0.005 µM (2.1t0.1-fold) |

| | | |
|---|---|---|
| ^{a} Nurr1 modulation was determined in a Gal4-Nurr1 hybrid reporter gene assay. Max. activation refers to the maximum effect vs. 0.1% DMSO control. Data are the mean±SD; n≥3. | | |

Potent Nurr1 agonism of **36** was also evident on full-length human Nurr1 (Figure 2a,b). **36** robustly activated the Nurr1 homodimer (NurRE, EC₅₀ = 0.094 µM; Figure 2a) and the Nurr1-RXR heterodimer (DR5, EC₅₀ = 0.165 µM; Figure 2b) but interestingly was inactive on the Nurr1 monomer (NBRE; Figure 2a) indicating an unprecedented Nurr1 dimer preference. The RXR agonist bexarotene caused generally reduced activity of DR5 but enhanced the potency of 36 by a factor of >5 (EC₅₀ = 0.033 µM (DR5 w. 0.1 µM BEX)) suggesting potentially cooperative binding³¹ to and activation of the Nurr1-RXR heterodimer.

**36** exhibited high affinity binding (K_{d} 0.17 µM) to the Nurr1 LBD in ITC (Figure 2c) orthogonally validating its potent Nurr1 agonism. Moreover, **36** induced expression of the Nurr1-regulated tyrosine hydroxylase (TH), vesicular amino acid transporter 2 (VMAT2), and superoxide dismutase 2 (SOD2) in astrocytes (T98G) at low concentrations (0.1 µM, 0.3 µM) supporting cellular target engagement. Selectivity profiling revealed a preference of **36** for Nurr1 over Nur77 (Table 6) and no activity outside the NR4A family at 3 µM corresponding to >30-fold selectivity (Figure 2e) and emphasizing **36** as a highly optimized Nurr1 agonist also fulfilling community agreed quality criteria for chemical tools^{32,33}. With this favorable profile, ³⁶ emerges as next-generation tool to study the effects of Nurr1 modulation by AQ-type ligands.

To boost the value of **36** as a chemical tool, we aimed to complement it with a structurally matched negative control compound for which **29** appeared suitable. **29** strongly resembles **36** in its chemical structure and physicochemical characteristics but exhibited no Nurr1 agonism in cellular setting at 10 µM and revealed no detectable binding to Nurr1 in ITC (Table 6). Further profiling of **29** revealed no effect on Nur77 and NOR-1, no activation of full-length Nurr1, and no induction of Nurr1-regulated gene expression (Figure 2d). Thus, **29** is more than 100-fold less active than **36** as Nurr1 modulator and suitable as negative control.

**Table 6. Characterization of NR4A agonist 36 and negative control 29**

| | 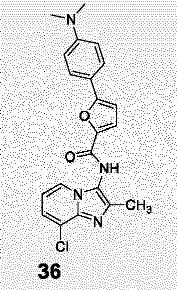 | 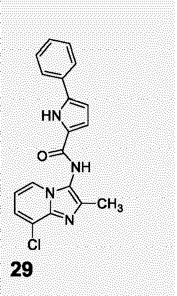 |
|---|---|---|
| EC₅₀(Nurr1) | 0.090±0.005 µM | no activation (10 µM) |
| EC₅₀(Nur77) | 0.33±0.04 µM | no activation (10 µM) |
| EC₅₀(NOR-1) | 0.1 1±0.03 µM | no activation (10 µM) |
| EC₅₀(NBRE) | no activation (1 µM) | no activation (10 µM) |
| EC₅₀(NurRE) | 0.094±0.003 µM | no activation (10 µM) |
| EC₅₀(DR5) [+0.1 µM BEX] | 0.165±0.005 µM [0.033±0.008 µM] | no activation (10 µM) |
| K_{d}(Nurr1 LBD) | 0.17 µM | no binding ^{b} |
| aq. solubility | 6.8 mg/L | 4.1 mg/L |
| SlogP ^{c} | 4.87 | 4.54 |

| | | |
|---|---|---|
| ^{a} Nurr1 modulation was determined in a Gal4-Nurr1 hybrid reporter gene assay. Max. activation refers to the maximum effect vs. 0.1% DMSO control. Data are the mean±SD; n≥3. ^{b} No binding observable in ITC with 100 µM **29** and 30 µM protein (Figure S2). ^{c} SlogP was computed with RDKit³⁴ software. | | |

### 3. Conclusion

Nurr1 is attracting remarkable interest as candidate target for neurodegenerative disease treatment¹². Therapeutic potential of the nuclear receptor is strongly supported by knockout studies and observations from patients¹². AQ (**1**) was discovered as direct Nurr1 modulator and used in pharmacological studies^{24,35} but is a weak Nurr1 agonist and exhibits non-specific effects¹⁹ disqualifying the antimalarial as Nurr1 agonist drug. We have developed potent and fully validated Nurr1 ligands from AQ (**1**). Scaffold-hopping from the chloroquinoline to chloroimidazopyridine and replacement of the aminophenol motif of AQ which has PAINS³⁶ character generated potent Nurr1 agonists with favorable properties and validated high-affinity binding to advance Nurr1 modulation as therapeutic strategy in neurodegeneration and beyond.

### REFERENCES

(1) Zetterström, R. H.; Williams, R.; Perlmann, T.; Olson, L. Cellular Expression of the Immediate Early Transcription Factors Nurr1 and NGFI-B Suggests a Gene Regulatory Role in Several Brain Regions Including the Nigrostriatal Dopamine System. Mol. Brain Res. 1996, 41 (1-2), 111-120.
(2) Kummari, E.; Guo-Ross, S. X.; Partington, H. S.; Nutter, J. M.; Eells, J. B. Quantitative Immunohistochemistry to Measure Regional Expression of Nurr1 in the Brain and the Effect of the Nurr1 Heterozygous Genotype. Front. Neuroanat. 2021, 15.
(3) Saijo, K.; Winner, B.; Carson, C. T.; Collier, J. G.; Boyer, L.; Rosenfeld, M. G.; Gage, F. H.; Glass, C. K. A Nurr1/CoREST Pathway in Microglia and Astrocytes Protects Dopaminergic Neurons from inflammation-induced Death. Cell 2009, 137 (1), 47-59.
(4) Zetterström, R. H.; Solomin, L.; Jansson, L.; Hoffer, B. J.; Olson, L.; Perlmann, T. Dopamine Neuron Agenesis in Nurr1-Deficient Mice. Science 1997, 276 (5310), 248-250.
(5) Kadkhodaei, B.; Ito, T.; Joodmardi, E.; Mattsson, B.; Rouillard, C.; Carta, M.; Muramatsu, S. I.; Sumi-ichinose, C.; Nomura, T.; Metzger, D.; Chambon, P.; Lindqvist, E.; Larsson, N. G.; Olson, L.; Björklund, A.; Ichinose, H.; Perlmann, T. Nurr1 Is Required for Maintenance of Maturing and Adult Midbrain Dopamine Neurons. J. Neurosci. 2009, 29 (50), 15923-15932.
(6) Willems, S.; Marschner, J.; Kilu, W.; Faudone, G.; Busch, R.; Duensing-Kropp, S.; Heering, J.; Merk, D. Nurr1 Modulation Mediates Neuroprotective Effects of Statins. Adv. Sci. 2022, e2104640.
(7) Chu, Y; Le, W.; Kompoliti, K.; Jankovic, J.; Mufson, E. J.; Kordower, J. H. Nurr1 in Parkinson's Disease and Related Disorders. J. Comp. Neurol. 2006, 494 (3), 495-514.
(8) Decressac, M.; Kadkhodaei, B.; Mattsson, B.; Laguna, A.; Perlmann, T.; Björklund, A. α-Synuclein-Induced down-Regulation of Nurr1 Disrupts GDNF Signaling in Nigral Dopamine Neurons. Sci. Transl. Med. 2012, 4 (163), 163ra156.
(9) Liu, W.; Gao, Y; Chang, N. Nurr1 Overexpression Exerts Neuroprotective and Anti-Inflammatory Roles via down-Regulating CCL2 Expression in Both in Vivo and in Vitro Parkinson's Disease Models. Biochem. Biophys. Res. Commun. 2017, 482 (4), 1312-1319.
(10) Parra-Damas, A.; Valero, J.; Chen, M.; España, J.; Martin, E.; Ferrer, I.; Rodríguez-Alvarez, J.; Saura, C. A. Crtc1 Activates a Transcriptional Program Deregulated at Early Alzheimer's Disease-Related Stages. J. Neurosci. 2014, 34 (17), 5776-5787.
(11) Yao, P. L.; Parmar, V. M.; Choudhary, M.; Malek, G. NURR1 Expression Regulates Retinal Pigment Epithelial-Mesenchymal Transition and Age-Related Macular Degeneration Phenotypes. Proc. Natl. Acad. Sci. U. S. A. 2022, 119 (28).
(12) Willems, S.; Merk, D. Medicinal Chemistry and Chemical Biology of Nurr1 Modulators: An Emerging Strategy in Neurodegeneration. J. Med. Chem. 2022, 65 (14), 9548-9563.
(13) Wang, Z.; Benoit, G.; Liu, J.; Prasad, S.; Aarnisalo, P.; Liu, X.; Xu, H.; Walker, N. P. C.; Perlmann, T. Structure and Function of Nurr1 Identifies a Class of Ligand-Independent Nuclear Receptors. Nature 2003, 423 (6939), 555-560.
(14) Willems, S.; Kilu, W.; Ni, X.; Chaikuad, A.; Knapp, S.; Heering, J.; Merk, D. The Orphan Nuclear Receptor Nurr1 Is Responsive to Non-Steroidal Anti-Inflammatory Drugs. Commun. Chem. 2020, 3, 85.
(15) Yu, X.; Shang, J.; Kojetin, D. J. Molecular Basis of Ligand-Dependent Nurr1-RXRα Activation. Elife 2023, 12.
(16) Bruning, J. M.; Wang, Y; Oltrabella, F.; Tian, B.; Kholodar, S. A.; Liu, H.; Bhattacharya, P.; Guo, S.; Holton, J. M.; Fletterick, R. J.; Jacobson, M. P.; England, P. M. Covalent Modification and Regulation of the Nuclear Receptor Nurr1 by a Dopamine Metabolite. Cell Chem. Biol. 2019, 26 (5), 674-685.e6.
(17) de Vera, I. M. S.; Giri, P. K.; Munoz-Tello, P.; Brust, R.; Fuhrmann, J.; Matta-Camacho, E.; Shang, J.; Campbell, S.; Wilson, H. D.; Granados, J.; Gardner, W. J. J.; Creamer, T. P.; Solt, L. A.; Kojetin, D. J. Identification of a Binding Site for Unsaturated Fatty Acids in the Orphan Nuclear Receptor Nurr1. ACS Chem. Biol. 2016, 11 (7), 1795-1799.
(18) Rajan, S.; Jang, Y; Kim, C. H.; Kim, W.; Toh, H. T.; Jeon, J.; Song, B.; Serra, A.; Lescar, J.; Yoo, J. Y; Beldar, S.; Ye, H.; Kang, C.; Liu, X. W.; Feitosa, M.; Kim, Y; Hwang, D.; Goh, G.; Lim, K. L. et al. PGE1 and PGA1 Bind to Nurr1 and Activate Its Transcriptional Function. Nat. Chem. Biol. 2020, 16 (8), 876-886.
(19) Munoz-Tello, P.; Lin, H.; Khan, P.; De Vera, I. M. S.; Kamenecka, T. M.; Kojetin, D. J. Assessment of NR4A Ligands That Directly Bind and Modulate the Orphan Nuclear Receptor Nurr1. J. Med. Chem. 2020, 63 (24), 15639-15654.
(20) Vietor, J.; Gege, C.; Stiller, T.; Busch, R.; Schallmayer, E.; Kohlhof, H.; Höfner, G.; Pabel, J.; Marschner, J. A.; Merk, D. Development of a Potent Nurr1 Agonist Tool for in Vivo Applications. J. Med. Chem. 2023, 66, 10.1021/acs.jmedchem.3c00415.
(21) Ballarotto, M.; Willems, S.; Stiller, T.; Nawa, F.; Marschner, J. A.; Grisoni, F.; Merk, D. De Novo Design of Nurr1 Agonists via Fragment-Augmented Generative Deep Learning in Low-Data Regime . J. Med. Chem. 2023, 66 (12), 8170-8177.
(22) Sai, M.; Vietor, J.; Kornmayer, M.; Egner, M.; López-García, U.; Höfner, G.; Pabel, J.; Marschner, J. A.; Wein, T.; Merk, D. Structure-Guided Design of Nurr1 Agonists Derived from the Natural Ligand Dihydroxyindole. J. Med. Chem. 2023, 66 (19), 13556-13567.
(23) Stiller, T.; Merk, D. Exploring Fatty Acid Mimetics as NR4A Ligands. J. Med. Chem. 2023.
(24) Kim, C.-H.; Han, B.-S.; Moon, J.; Kim, D.-J.; Shin, J.; Rajan, S.; Nguyen, Q. T.; Sohn, M.; Kim, W.-G.; Han, M.; Jeong, I.; Kim, K.-S.; Lee, E.-H.; Tu, Y; Naffin-Olivos, J. L.; Park, C.-H.; Ringe, D.; Yoon, H. S.; Petsko, G. A. et al. Nuclear Receptor Nurr1 Agonists Enhance Its Dual Functions and Improve Behavioral Deficits in an Animal Model of Parkinson's Disease. Proc. Natl. Acad. Sci. 2015, 112 (28), 8756-8761.
(25) Moon, M.; Jung, E. S.; Jeon, S. G.; Cha, M.-Y; Jang, Y; Kim, W.; Lopes, C.; Mook-Jung, I.; Kim, K.-S. Nurr1 (NR4A2) Regulates Alzheimer's Disease-Related Pathogenesis and Cognitive Function in the 5XFAD Mouse Model. Aging Cell 2019, 18, e12866.
(26) Kim, W.; Tripathi, M.; Kim, C.; Vardhineni, S.; Cha, Y; Kandi, S. K.; Feitosa, M.; Kholiya, R.; Sah, E.; Thakur, A.; Kim, Y; Ko, S.; Bhatia, K.; Manohar, S.; Kong, Y.-B.; Sindhu, G.; Kim, Y.-S.; Cohen, B.; Rawat, D. S. et al. An Optimized Nurr1 Agonist Provides Disease-Modifying Effects in Parkinson's Disease Models. Nat. Commun. 2023, 14 (1), 4283.
(27) Willems, S.; Ohrndorf, J.; Kilu, W.; Heering, J.; Merk, D. Fragment-like Chloroquinolineamines Activate the Orphan Nuclear Receptor Nurr1 and Elucidate Activation Mechanisms. J. Med. Chem. 2021, 64 (5), 2659-2668.
(28) Willems, S.; Müller, M.; Ohrndorf, J.; Heering, J.; Proschak, E.; Merk, D. Scaffold Hopping from Amodiaquine to Novel Nurr1 Agonist Chemotypes via Microscale Analogue Libraries. ChemMedChem 2022, 17 (8), e2022000.
(29) Hopkins, A. L.; Keserü, G. M.; Leeson, P. D.; Rees, D. C.; Reynolds, C. H. The Role of Ligand Efficiency Metrics in Drug Discovery. Nat. Rev. Drug Discov. 2014, 13 (2), 105-121.
(30) Willems, S.; Müller, M.; Ohrndorf, J.; Heering, J.; Proschak, E.; Merk, D. Scaffold Hopping from Amodiaquine to Novel Nurr1 Agonist Chemotypes via Microscale Analogue Libraries. ChemMedChem 2022, 17 (8), e2022000.
(31) de Vink, P. J.; Koops, A. A.; D'Arrigo, G.; Cruciani, G.; Spyrakis, F.; Brunsveld, L. Cooperativity as Quantification and Optimization Paradigm for Nuclear Receptor Modulators. Chem. Sci. 2022, 13 (9), 2744-2752.
(32) Hartung, I. V.; Rudolph, J.; Mader, M. M.; Mulder, M. P. C.; Workman, P. Expanding Chemical Probe Space: Quality Criteria for Covalent and Degrader Probes. J. Med. Chem. 2023, 66 (14), 9297-9312.
(33) https://www.thesgc.org/chemical-probes/.
(34) RDKit: Open-source cheminformatics. Open-Source Cheminformatics. *http:*//*www.rdkit.org.*
(35) Moon, M.; Jung, E. S.; Jeon, S. G.; Cha, M.-Y; Jang, Y; Kim, W.; Lopes, C.; Mook-Jung, I.; Kim, K.-S. Nurr1 (NR4A2) Regulates Alzheimer's Disease-Related Pathogenesis and Cognitive Function in the 5XFAD Mouse Model. Aging Cell 2019, 18 (1), e12866.
(36) Baell, J. B.; Nissink, J. W. M. Seven Year Itch: Pan-Assay Interference Compounds (PAINS) in 2017-Utility and Limitations. ACS Chem. Biol. 2017, 13 (1), 36-44.
(37) Flesch, D.; Cheung, S.-Y; Schmidt, J.; Gabler, M.; Heitel, P.; Kramer, J. S.; Kaiser, A.; Hartmann, M.; Lindner, M.; Lüddens-Dämgen, K.; Heering, J.; Lamers, C.; Lüddens, H.; Wurglics, M.; Proschak, E.; Schubert-Zsilavecz, M.; Merk, D. Non-Acidic Farnesoid X Receptor Modulators. J. Med. Chem. 2017, 60 (16), 7199-7205.
(38) Pauli, G. F.; Chen, S. N.; Simmler, C.; Lankin, D. C.; Gödecke, T.; Jaki, B. U.; Friesen, J. B.; McAlpine, J. B.; Napolitano, J. G. Importance of Purity Evaluation and the Potential of Quantitative 1H NMR as a Purity Assay. J. Med. Chem. 2014, 57 (22), 9220-9231.
(39) Gellrich, L.; Heitel, P.; Heering, J.; Kilu, W.; Pollinger, J.; Goebel, T.; Kahnt, A.; Arifi, S.; Pogoda, W.; Paulke, A.; Steinhilber, D.; Proschak, E.; Wurglics, M.; Schubert-Zsilavecz, M.; Chaikuad, A.; Knapp, S.; Bischoff, I.; Fürst, R.; Merk, D. L-Thyroxin and the Nonclassical Thyroid Hormone TETRAC Are Potent Activators of PPARγ. J. Med. Chem. 2020, 63 (13), 6727-6740.
(40) Pollinger, J.; Gellrich, L.; Schierle, S.; Kilu, W.; Schmidt, J.; Kalinowsky, L.; Ohrndorf, J.; Kaiser, A.; Heering, J.; Proschak, E.; Merk, D. Tuning Nuclear Receptor Selectivity of Wy14,643 towards Selective Retinoid X Receptor Modulation. J. Med. Chem. 2019, 62 (4), 2112-2126.
(41) Rau, O.; Wurglics, M.; Paulke, A.; Zitzkowski, J.; Meindl, N.; Bock, A.; Dingermann, T.; Abdel-Tawab, M.; Schubert-Zsilavecz, M. CarnosicAcid and Carnosol, Phenolic Diterpene Compounds of the Labiate Herbs Rosemary and Sage, Are Activators of the Human Peroxisome Proliferator-Activated Receptor Gamma. Planta Med. 2006, 72 (10), 881-887.
(42) Schmidt, J.; Klingler, F.-M.; Proschak, E.; Steinhilber, D.; Schubert-Zsilavecz, M.; Merk, D. NSAIDs Ibuprofen, Indometacin, and Diclofenac Do Not Interact with Farnesoid X Receptor. Sci. Rep. 2015, 5, 14782.
(43) Heitel, P.; Gellrich, L.; Kalinowsky, L.; Heering, J.; Kaiser, A.; Ohrndorf, J.; Proschak, E.; Merk, D. Computer-Assisted Discovery and Structural Optimization of a Novel Retinoid X Receptor Agonist Chemotype. ACS Med. Chem. Lett. 2019, 10 (2), 203-208.
(44) Seuter, S.; Väisänen, S.; Rådmark, O.; Carlberg, C.; Steinhilber, D. Functional Characterization of Vitamin D Responding Regions in the Human 5-Lipoxygenase Gene. Biochim. Biophys. Acta 2007, 1771 (7), 864-872.

## Claims

1. A compound of formula (I) or a salt or solvate thereof: 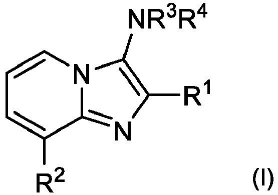 wherein
R¹ is selected from -H, -OH, -C₁-C₄ alkyl optionally substituted, -C₂-C₄ alkenyl optionally substituted, -C₂-C₄ alkynyl optionally substituted, -C₃-C₈ cycloalkyl optionally substituted, -OC₁-C₄ alkyl optionally substituted, -OC₂-C₄ alkenyl optionally substituted, -OC₂-C₄ alkynyl optionally substituted, -OC₃-C₈ cycloalkyl optionally substituted, phenyl optionally substituted, heteroaryl optionally substituted, -O-phenyl optionally substituted or -O-heteroaryl optionally substituted,
R² is selected from halo, -C₁-C₄ alkyl optionally substituted, -C₂-C₄ alkenyl optionally substituted, C₂-C₄ alkynyl optionally substituted -OC₁-C₄ alkyl optionally substituted, -OC₂-C₄ alkenyl optionally substituted, -OC₂-C₄ alkynyl optionally substituted, -C₃-C₈ cycloalkyl optionally substituted, or -OC₃-C₈ cycloalkyl optionally substituted,
R³ is selected from H or -CH₃ optionally substituted,
R⁴ is selected from H, -CH₃ optionally substituted or -R⁵,
R⁵ is 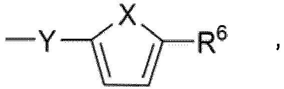
X is selected from O, S or NR¹¹,
Y is selected from -C(O)- or C₁-C₃- alkylene,
R⁶ is selected from phenyl, heteroaryl, C₃-C₈ cycloalkyl, C₁-C₆ alkyl, -C₂-C₆ alkenyl optionally substituted, -C₂-C₆ alkynyl optionally substituted, wherein each of said residues is optionally substituted with -R⁷, -OR⁸, -NR⁹R¹⁰ and/or halo;
R⁷ is selected from -H, halo, -C₁-C₆ alkyl optionally substituted, -C₂-C₆ alkenyl optionally substituted, -C₂-C₆ alkynyl optionally substituted, or -C₃-C₈ cycloalkyl optionally substituted,
R⁸ is selected from H, halo, C₁-C₆ alkyl optionally substituted, -C₂-C₆ alkenyl optionally substituted, -C₂-C₆ alkynyl optionally substituted or C₃-C₈ cycloalkyl optionally substituted,
R⁹ and R¹⁰ are independently selected from H, halo, C₁-C₆ alkyl optionally substituted, -C₂-C₆ alkenyl optionally substituted, -C₂-C₆ alkynyl optionally substituted or C₃-C₈ cycloalkyl optionally substituted or wherein R⁹ and R¹⁰ may form a cyclic system, and
R¹¹ is selected from H, C₁-C₆ alkyl optionally substituted, -C₂-C₆ alkenyl optionally substituted, -C₂-C₆ alkynyl optionally substituted or C₃-C₈ cycloalkyl optionally substituted.

2. The compound of claim 1 wherein (i) R¹ is -CH³, (ii) R² is CI, and/or (iii) R³ is H.

3. The compound of claim 1 or 2 wherein R⁵ is:

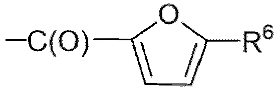

4. The compound of any one of claims 1-3 wherein R⁶ is phenyl substituted at position 4 relative to the position of attachment to the basic structure.

5. The compound of any one of claims 1-4
wherein R⁶ is phenyl substituted with -R⁷, -OR⁸, -NR⁹R¹⁰ and/or halo,
wherein R⁷ is -CH₃ optionally substituted with -F, particularly -CH₃ or -CF₃;
wherein R⁸ is -CH₃ optionally substituted with -F, particularly -CH₃ or -CF₃, and
wherein R⁹ and R¹⁰ are independently selected from H and -CH₃ optionally substituted with -F.

6. The compound of any one of claims 1-5 wherein R⁹ and R¹⁰ are -CH₃.

7. The compound of any one of claims 1-6 which is 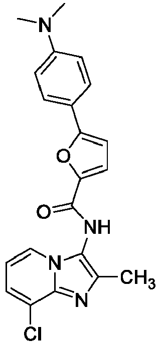 or a salt or solvate thereof.

8. A pharmaceutical composition comprising the compound of formula (I) or a salt or solvate thereof of any one of claims 1-7 as an active agent and a pharmaceutically acceptable carrier.

9. A compound of formula (I) or a salt or solvate of any one of claims 1-7 or a pharmaceutical composition of claim 8 for use in medicine.

10. A compound of formula (I) or a salt or solvate of any one of claims 1-7 or a pharmaceutical composition of claim 8 for use in the prevention and/or treatment of a disease caused by and/or associated with insufficient Nurr1 activity.

11. A compound of formula (I) or a salt or solvate of any one of claims 1-7 or a pharmaceutical composition of claim 8 for use in the prevention and/or treatment of a degenerative disease, an inflammatory disease and/or a hyperproliferative disease.

12. A compound of formula (I) or a salt or solvate thereof for use in the prevention and/or treatment of a neurodegenerative disease, an ocular disease, a gastro-intestinal disease, or cancer caused by and/or associated with insufficient Nurr1 activity.

13. A compound of formula (I) or a salt or solvate of any one of claims 1-7 or a pharmaceutical composition of claim 8 for use in the prevention and/or treatment of a disease selected from Morbus Alzheimer, Morbus Parkinson, dementia, multiple sclerosis, progressive supranuclear palsy (PSP), ischemic stroke, schizophrenia, depression, ADHD, circadian rhythm disorder, rheumatoid arthritis, lupus erythematosus, or inflammatory bowel disease, or macular degeneration including age-related macular degeneration.

14. A compound of formula (I) or a salt or solvate of any one of claims 1-7 or a pharmaceutical composition of claim 8 for use of any one of claims 9-13 as a monotherapy or as a combination therapy with at least one further medicament.

15. A method of preventing and/or treating disease caused by and/or associated with insufficient Nurr1 activity comprising administering to a subject in need thereof an effective amount of a compound of formula (I).
